# EUROPEAN PATENT APPLICATION

(11) **EP 4 122 401 A1**
(43) Date of publication of application: **25.01.2023**
(21) Application number: 21771663.8
(22) Date of filing: 10.03.2021
(51) Int. Cl.: A61B 17/00, A61B 17/12

(54) **BIODEGRADABLE OCCLUDER OF PARACHUTE DESIGN**

(30) Priority: 15.03.2020 CN 202010179058; 15.03.2020 CN 202020319917 U
(71) Applicant: Shanghai Shape Memory Alloy Co., Ltd., Shanghai, 201612 (CN)
(72) Inventor: CHEN, Juan, Shanghai 201612 (CN); WANG, Yunbing, Shanghai 201612 (CN); WANG, Fan, Shanghai 201612 (CN); HU, Jinpeng, Shanghai 201612 (CN); XU, Xianchun, Shanghai 201612 (CN)
(74) Representative: Cabinet Beau de Loménie
(86) International application number: PCT/CN2021/079880
(87) International publication number: WO 2021/185127

(57) **Abstract**

The present invention provides a parachute-shaped biodegradable occluder, with a proximal end adjacent to a human body and a distal end far away from the human body. The parachute-shaped biodegradable occluder includes an occluder stent internally provided with a baffle film, and a shapeable ring with changeable states. The occluder stent, the baffle film and the shapeable ring are respectively made of polymer materials being degradable in biological tissues. The occluder is made of a biodegradable polymer material, which is degradable or absorbable by human tissues after being implanted for a period of time to make a defect site composed of human tissues, realizing occlusion without foreign matter. The occlusion effect is particularly significant for children.

## Description

### TECHNICAL FIELD

The present invention relates to a biodegradable occluder.

### BACKGROUND

Common congenital heart defects (CHDs) include atrial septal defect (ASD), ventricular septal defect (VSD), patent ductus arteriosus (PDA) and patent foramen ovale (PFO). In the past, surgical thoracotomy was the only effective way to treat CHDs. With the development of interventional medical equipment and interventional surgery technology, the previous surgical thoracotomy is replaced by the minimally invasive interventional therapy to cure the patients with CHDs. Conventional minimally invasive interventional surgery uses a cardiac occluder for occlusion treatment. At present, the mainstream occluder on the market is made of nickel-titanium alloy material. The nickel-titanium alloy material has super-elastic performance to achieve the occlusion treatment.

The use of the conventional metal occluder will cause complications in a certain proportion. Since the nickel-titanium alloy material is not degradable, the occluder will permanently exist in the heart after being implanted in the human body, which limits other later treatments, such as occlusion of the left atrial appendage (LAA). For occlusion of VSD, the use of the conventional metal occluder is likely to cause a conduction block in a certain probability. After the occluder is implanted in the human body, due to the hard material, it may rub against the defect tissue and surrounding tissues to cause tissue damage.

### SUMMARY

An objective of the present invention is to provide an occluder, which achieves an occlusion effect in a short time, and is degradable or absorbable by tissues after long-term endothelialization.

In order to achieve the above objective, the present invention provides the following technical solution: a parachute-shaped biodegradable occluder, with a proximal end adjacent to a human body and a distal end far away from the human body, where the occluder includes an occluder stent internally provided with a baffle film, and a shapeable ring with changeable states; and the occluder stent, the baffle film and the shapeable ring are respectively made of polymer materials being degradable in biological tissues;
a distal end of the occluder stent defines a distal disc surface, and a proximal end of the occluder stent defines a proximal disc surface; a part of the occluder stent located between the distal disc surface and the proximal disc surface defines an occluder waist; the proximal end of the occluder stent is provided with a joint; and the shapeable ring enters the occluder stent through a through hole of the joint; and
the states of the shapeable ring are changeable between a contracted state and an expanded state; when the shapeable ring is unconstrained or unstressed, the shapeable ring is in the expanded state, and the expanded shapeable ring is located inside the occluder stent; at this time, the occluder is not shaped, and is deliverable in a slender delivery sheath; when an external force is applied, the shapeable ring changes from the expanded state to the contracted state; the shapeable ring in the contracted state is allowed to pass through the through hole of the joint on the occluder stent; when the external force is removed, the shapeable ring is restored to the expanded state; since the occluder stent has a certain resilience and the shapeable ring plays a role of auxiliary shaping, the deformed distal disc surface of the occluder stent is restored to a designed state; and a proximal end of the shapeable ring has a proximal structure to facilitate the application of the external force to the shapeable ring; a distal end of the shapeable ring is provided with M connection lines, M>_3; the M connection lines are connected and fixed to the distal disc surface of the occluder stent; M connection points of the M connection lines and the distal disc surface are located on or within a projection circle with a diameter of the occluder waist; and when a force is applied to the proximal structure of the shapeable ring, the force is evenly distributed to each of the connection points through the connection lines at the distal end of the shapeable ring, such that the occluder waist is pulled and fastened, and the distal disc surface and the proximal disc surface are brought into contact with each other.

Preferably, the baffle film may be a single-layer baffle film or an N-layer baffle film, N≥2.

Preferably, when a force may be applied to the proximal structure of the shapeable ring, the shapeable ring may drive a center of the distal disc surface to be concave, and an inner side of the distal disc surface may squeeze the occluder waist to strengthen the support of the occluder waist.

Preferably, the shapeable ring may be constrained to the contracted state through the through hole of the joint on the occluder stent to allow the biodegradable occluder to be entirely retrievable.

The parachute-shaped biodegradable occluder provided by the present invention has the following advantages:
1) The occluder is made of a biodegradable polymer material, which is degradable or absorbable by human tissues after being implanted for a period of time to make a defect site composed of human tissues, realizing occlusion without foreign matter. The occlusion effect is particularly significant for children.
2) Due to the appropriate material selected, the occluder of the present invention is softer than the metal occluder, and has less friction and damage to the defect tissue and surrounding tissues after being implanted in the human body.
3) The special design of the shapeable ring of the occluder can achieve expected shapes of the left and right discs of the occluder, which compensates for the defect of poor resilience of the material of the occluder, and make the occluder fit the defect tissue smoothly to achieve the occlusion effect.
4) The occluder provided by the present invention is retrievable, which can deal with abnormal situations that may occur clinically, and realize the optimal treatment for the patient.
5) The occluder provided by the present invention is simple to operate, and compared with the metal occluder, it achieves better occlusion treatment without increasing the complexity of the operation.

### BRIEF DESCRIPTION OF THE DRAWINGS

FIG. 1 is a structural view of a parachute-shaped biodegradable occluder according to the present invention;

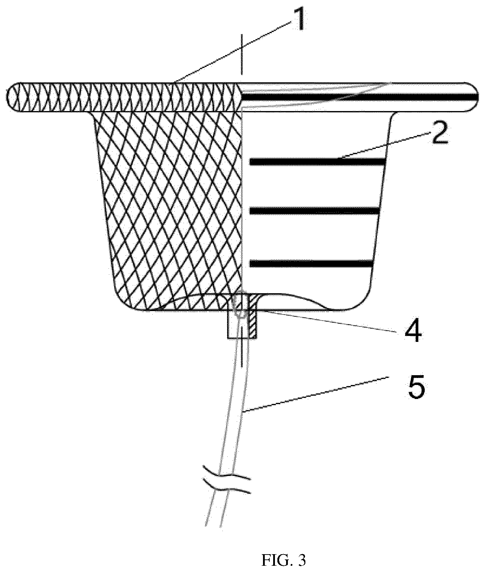

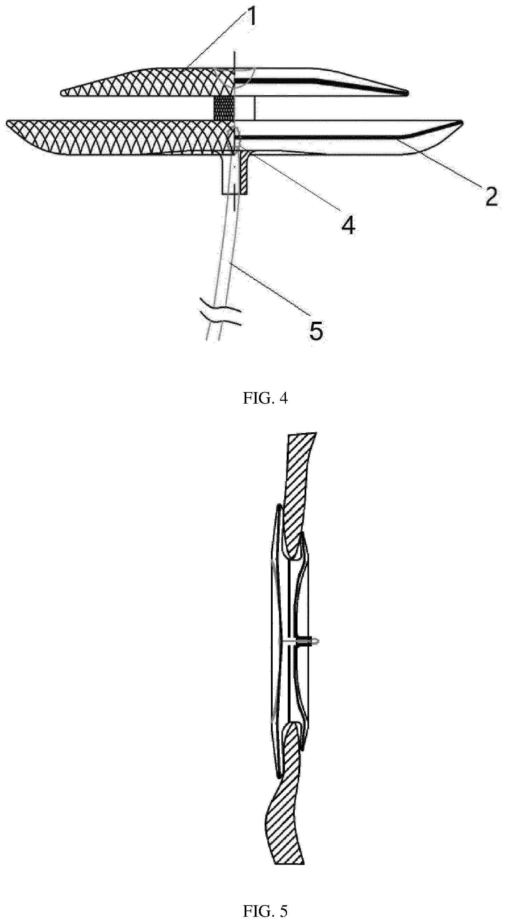

FIGS. 2 to 4 show parachute-shaped biodegradable occluders in different structural forms;
FIG. 5 shows the parachute-shaped biodegradable occluder shaped at a defect site according to the present invention;
FIGS. 6A and 6B show a shapeable ring adopting a first wiring form;
FIGS. 7A and 7B show a shapeable ring adopting a second wiring form;
FIGS. 8A and 8B show a shapeable ring adopting a third wiring form; and

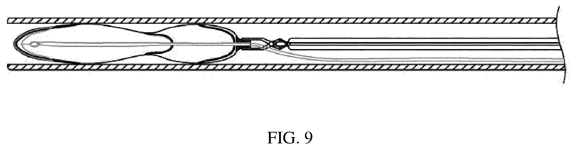

FIG. 9 shows the parachute-shaped biodegradable occluder in a delivery sheath according to the present invention.

### DETAILED DESCRIPTION OF THE EMBODIMENTS

The present invention will be described in detail below with reference to specific embodiments. It should be understood that these examples are only intended to describe the present invention, rather than to limit the scope of the present invention. In addition, it should be understood that various changes and modifications may be made on the present invention by those skilled in the art after reading the content of the present invention, and these equivalent forms also fall within the scope defined by the appended claims of the present invention.

The present invention provides a parachute-shaped biodegradable occluder. The occluder is made of a polymer material, which can be degraded into a harmless, absorbable or metabolizable substance in human biological tissues.

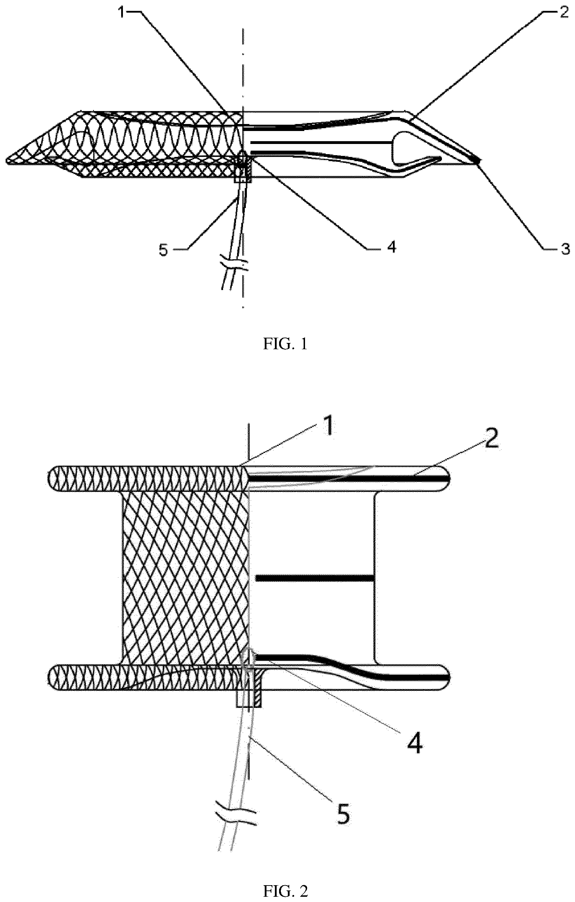

As shown in FIG. 1, the occluder includes an occluder stent 1, a baffle film 2 and a shapeable ring 4. The occluder stent 1 may be braided from a line, carved from a tube, or made by three-dimensional (3D) printing. An end of the occluder stent adjacent to a human body is defined as a proximal end, and an end of the occluder stent away from the human body is defined as a distal end. The distal end of the occluder stent 1 defines a distal disc surface, and the proximal end of the occluder stent defines a proximal disc surface. A part of the occluder stent located between the distal disc surface and the proximal disc surface defines an occluder waist. The proximal end of the occluder stent 1 is provided with a joint with a through hole.

In order to deliver the occluder provided by the present invention to a preset position, a development mark 3 is provided at an appropriate position on the occluder stent 1 or the baffle film 2. Through the development mark 3, the delivery path and position of the occluder provided by the present invention can be accurately grasped. The specific position of the development mark 3 is set by those skilled in the art as needed, and is not limited here.

The baffle film 2 is located in the occluder stent 1, and those skilled in the art may set a single-layer baffle film 2 or a multi-layer baffle film 2 as needed. The baffle film 2 is distributed at the proximal end and the distal end of the occluder stent 1, and the baffle film 2 at the proximal end is provided with a through hole.

The shapeable ring 4 is parachute-shaped, that is, the shapeable ring is round or elliptical as a whole when it is unconstrained or unstressed, which indicates an expanded state of the shapeable ring 4. A proximal end of the shapeable ring 4 is a ring structure. Like a parachute, the shapeable ring 4 can be folded to a contracted state or expanded to the expanded state. The ring structure of the shapeable ring 4 exerts an external force to the shapeable ring 4, such that the shapeable ring 4 changes from the expanded state to the contracted state. In this embodiment, the shapeable ring 4 is pulled by a shaping line 5 to apply an external force to the shapeable ring 4. The shapeable ring 4 in the contracted state is allowed to smoothly pass through the through hole of the joint at the proximal end of the occluder stent 1 and the through hole of the baffle film 2 at the proximal end. Due to the constraining effect of the through hole of the shapeable ring 4, the shapeable ring 4 always maintains the contracted state when passing through the through hole. After part or all of the shapeable ring 4 passes through the through hole, the shapeable ring is no longer constrained and is restored to an original shape. A distal end of the shapeable ring 4 is provided with connection lines, and the connection lines are connected to the distal disc surface of the occluder stent 1. The shapeable ring 4 and the distal disc surface are provided with multiple (more than 3) connection points, which are located on or within a projection circle with a diameter of the occluder waist. For example, the shapeable ring 4 is provided with four connection lines, and the connection points between the shapeable ring 4 and the distal disc surface are located on the projection circle with the diameter of the occluder waist. The shapeable ring 4 is provided with 8 connection lines, and the connection points between the shapeable ring 4 and the distal disc surface are located on a central ring line of the distal disc surface. The shapeable ring 4 is provided with 8 connection lines, and the connection points of the shapeable ring 4 and the distal disc surface are located on a projection circle with the diameter of the occluder waist, and the 8 connection lines are knitted in warp and weft. This design enhances the shaping effect of the connection lines and avoids entanglement and kinking between the connection lines.

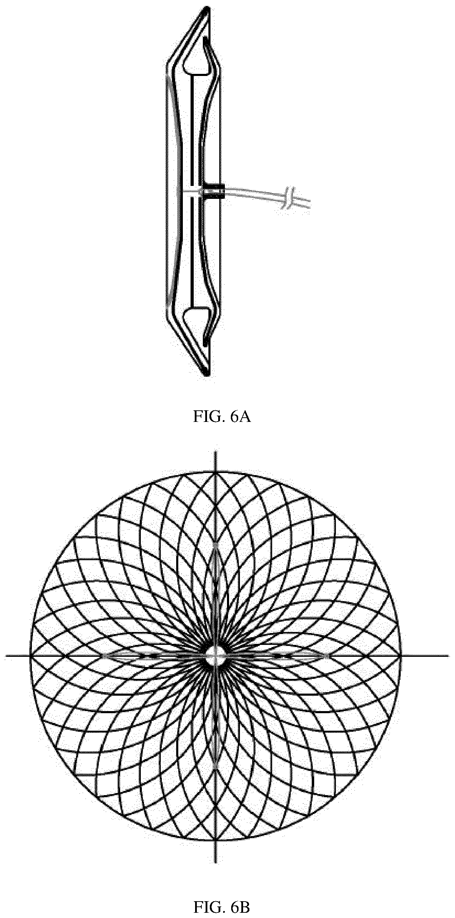

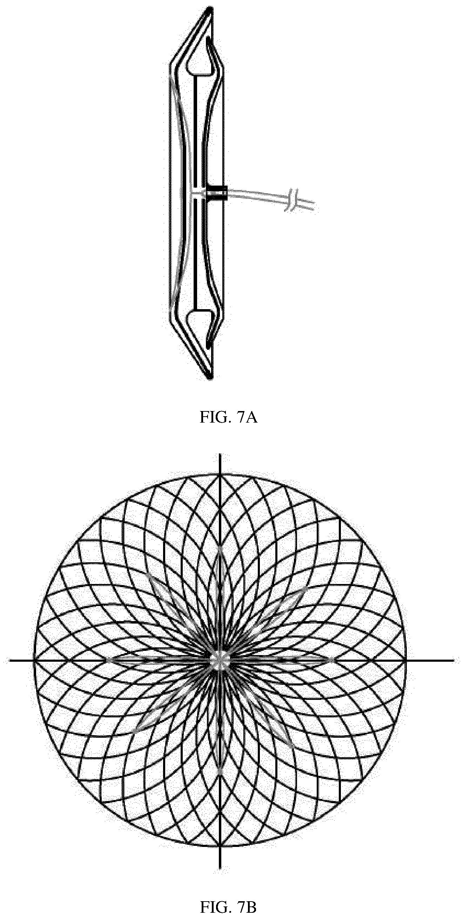

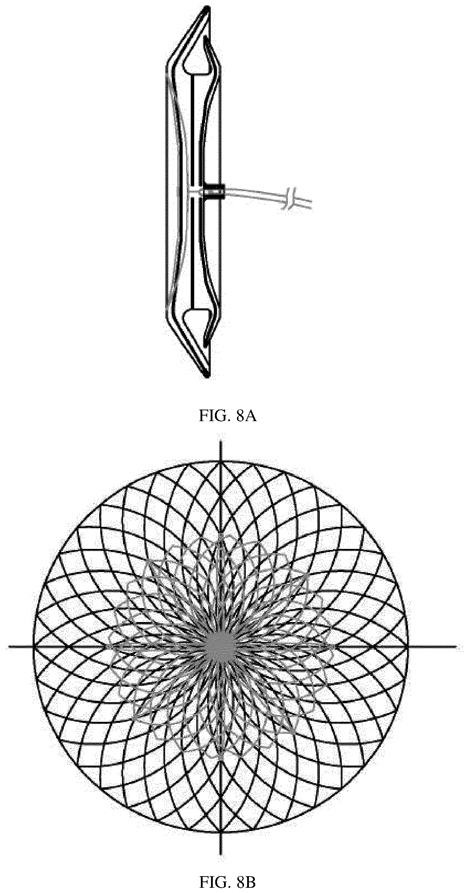

Those skilled in the art may select the wiring form of the shapeable ring 4 as needed. FIGS. 6A and 6B, 7A and 7B, 8A and 8B schematically show three different wiring forms. The wiring forms of the shapeable ring are fixing patterns of the shapeable ring and the occluder stent, as shown in FIGS. 6A, 6B, 7A, 7B, 8A and 8B, there are different braiding connection modes.

The occluder stent 1 is made of a polymer material, which has a poorer memory resilience than a metal material. Therefore, after the occluder stent 1 is delivered into a human body through a delivery sheath, the occluder stent 1 cannot be restored to a designed shape. In order to solve this problem, in the present invention, the shapeable ring 4 is implanted in the occluder stent 1. The shapeable ring 4 partially or all in the expanded state supports the occluder stent 1, and helps the occluder stent 1 to restore to a designed shape, thereby making up for the defect of poor memory resilience of the polymer material.

When a force is applied to the ring structure of the shapeable ring 4, the force is evenly dispersed to each of the connection points through the connection lines of the shapeable ring 4. In this way, the entire occluder waist is pulled and fastened, which maximally compensates for the defect of poor resilience of the material, so as to achieve an expected shape of the occluder.

This connection method can restore the distal disc surface of the occluder stent 1 to an expected shape, and avoid causing the distal disc surface to bulge or to adhere poorly to a defect site, which will result in thickening of the occluded site in a later stage. Meanwhile, the distal disc surface and the proximal disc surface are in close contact with each other, which can reduce the occurrence of residual shunts, and greatly improve the occlusion effect of mechanotherapy.

The connection method can also improve the support performance of the occluder waist. The shapeable ring 4 drives a center of the distal disc surface to be concave, and an inner side of the distal disc surface squeezes the occluder waist, thereby strengthening the support of the occluder waist and stably fixing the occluder.

This connection method can increase the clamping performance of the distal disc surface and the proximal disc surface. The connection points of the connection lines of the shapeable ring 4 are located on or within the projection circle with the diameter of the occluder waist. This increases the rigidity of the distal disc surface, and drives the distal disc surface and the proximal disc surface to fit each other, thereby increasing the clamping force of the two disc surfaces and stably fixing the occluder.

The occluder provided by the present invention is also retrievable. When the implanted occluder is not fully released and an immediate occlusion effect is observed by angiography, if the occlusion effect is not desirable or the selected occluder is not suitable, the occluder can be retrieved and replaced with a suitable one. When the occluder is implanted in the human body, the shapeable ring 4 passes through the through hole of the joint at the proximal end of the occluder stent 1 and is located outside the through hole or half stuck in the through hole. Then the elastic shapeable ring 4 is restored to the original round or elliptical shape. Thus, the occluder is completely shaped. The proximal disc surface and the distal disc surface are completely fastened, and smoothly fit the tissues of the defect site, so as to achieve a certain locking strength. When the occluder needs to be retrieved, a delivery device is not separated from the joint at the proximal end of the occluder stent 1. The delivery device is pulled, and when the pulling force is greater than the locking strength of the shapeable ring 4, the shapeable ring 4 is deformed to pass through the through hole of the joint. Due to the constraining effect of the through hole on the shapeable ring 4, the shapeable ring 4 remains in the contracted state and is pulled back into the occluder stent again. A withdrawal force is continuously applied to the delivery device until the entire occluder is pulled back into the delivery sheath and finally withdrawn from the human body.

## Claims

1. A parachute-shaped biodegradable occluder, with a proximal end adjacent to a human body and a distal end far away from the human body, comprising an occluder stent internally provided with a baffle film, and a shapeable ring with changeable states; and the occluder stent, the baffle film and the shapeable ring are respectively made of polymer materials being degradable in biological tissues;
a distal end of the occluder stent defines a distal disc surface, and a proximal end of the occluder stent defines a proximal disc surface; a part of the occluder stent located between the distal disc surface and the proximal disc surface defines an occluder waist; the proximal end of the occluder stent is provided with a joint; and the shapeable ring enters the occluder stent through a through hole of the joint; and
states of the shapeable ring are changeable between a contracted state and an expanded state; when the shapeable ring is unconstrained or unstressed, the shapeable ring is in the expanded state, and the occluder is not shaped and is deliverable in a slender delivery sheath; when an external force is applied, the shapeable ring changes from the expanded state to the contracted state; the shapeable ring in the contracted state is allowed to pass through the through hole of the joint on the occluder stent; when the external force is removed, the shapeable ring is restored to the expanded state; wherein the occluder stent has a certain resilience and the shapeable ring plays a role of auxiliary shaping, and a deformed distal disc surface of the occluder stent is restored to a designed state; a proximal end of the shapeable ring has a proximal structure to facilitate an application of the external force to the shapeable ring; a distal end of the shapeable ring is provided with M connection lines, M≥3; the M connection lines are connected and fixed to the distal disc surface of the occluder stent; M connection points of the M connection lines and the distal disc surface are located on or within a projection circle with a diameter of the occluder waist; and when a force is applied to the proximal structure of the shapeable ring, the force is evenly distributed to each of the connection points through the connection lines at the distal end of the shapeable ring, wherein the occluder waist is pulled and fastened, and the distal disc surface and the proximal disc surface are brought into contact with each other.

2. The parachute-shaped biodegradable occluder according to claim 1, wherein the baffle film is a single-layer baffle film or an N-layer baffle film, N≥2.

3. The parachute-shaped biodegradable occluder according to claim 1, wherein when a force is applied to the proximal structure of the shapeable ring, the shapeable ring drives a center of the distal disc surface to be concave, and an inner side of the distal disc surface squeezes the occluder waist to strengthen a support of the occluder waist.

4. The parachute-shaped biodegradable occluder according to claim 1, wherein the shapeable ring is constrained to the contracted state through the through hole of the joint on the occluder stent to allow the biodegradable occluder to be entirely retrievable.
